# EUROPÄISCHE PATENTANMELDUNG

(11) **EP 4 545 058 A1**
(43) Veröffentlichungstag der Anmeldung: **30.04.2025**
(21) Anmeldenummer: 24208154.5
(22) Anmeldetag: 22.10.2024
(51) Int. Cl.: A61H 7/00, A61F 13/08

(54) **BEKLEIDUNGSSTÜCK ZUM ANREGEN EINES LYMPHFLUSSES IN EINEM KÖRPERBEREICH EINES TRÄGERS**

(30) Priorität: 23.10.2023 DE 102023210404
(71) Anmelder: Möllenhoff, Christian, 90427 Nürnberg (DE)
(72) Erfinder: Möllenhoff, Christian, 90427 Nürnberg (DE)
(74) Vertreter: Rau, Schneck & Hübner Patentanwälte Rechtsanwälte PartGmbB

(57) **Zusammenfassung**

Bekleidungsstück (3, 12) zum Anregen eines Lymphflusses in einen Körperbereich (1, 11) eines Trägers (2) mit einem Grundkörper (4, 13) aufweisend eine Vielzahl an Vorsprüngen (8), welche an einer Innenseite (9) des Bekleidungsstücks (3, 12) angeordnet sind, wobei die Vorsprünge (8) derart angeordnet sind, dass diese mindestens eine Bahn (7, 16) ausbilden, wobei die mindestens eine Bahn (7, 16) entlang mindestens einer Lymphbahn eines Trägers (2) verläuft.

## Beschreibung

Der Inhalt der deutschen Patentanmeldung DE 10 2023 210 404.0 wird durch Bezugnahme hierin aufgenommen.

Die Erfindung betrifft ein Bekleidungsstück zum Anregen eines Lymphflusses in einem Körperbereich eines Trägers.

Personen mit Bindegewebsstörungen leiden unter anderem an einer Ansammlung von Flüssigkeiten in den Zwischenzellräumen des Bindegewebes. Bei Bindegewebserkrankungen, wie beispielsweise dem Lipödem, kann es unter Umständen zu Transportstörungen in genau diesem Gefäßanteil kommen, sodass im Gewebe ein Flüssigkeitsstau entsteht. Derartiger Flüssigkeitsstau wird mittels Lymphdrainage, welche durch spezielle Massagetechniken von ausgebildeten Physiotherapeuten angeregt wird, behandelt.

Es ist Aufgabe der vorliegenden Erfindung, ein Bekleidungsstück zu schaffen, welches den Lymphfluss von Personen anregt.

Die Aufgabe ist erfindungsgemäß durch ein Bekleidungsstück mit den in Anspruch 1 angegebenen Merkmalen gelöst.

Erfindungsgemäß wurde erkannt, dass der Lymphfluss durch ein Bekleidungsstück mit einer Vielzahl an Vorsprüngen, welche auf der Innenseite des Bekleidungsstücks angeordnet sind und entlang mindestens einer Bahn verlaufen, wobei die Bahn entlang mindestens einer Lymphbahn eines Trägers verläuft, effizient angeregt werden kann.

Sofern das Bekleidungsstück eine Vielzahl an Bahnen mit Vorsprüngen aufweist, können sich die Bahnen entlang ihres Verlaufs ein oder mehrmals schneiden.

Die Vorsprünge erfahren bei einer Bewegung des Trägers eine Mikrobewegung. Durch die Mikrobewegung der Vorsprünge wird der Lymphfluss in der jeweiligen zugeordneten Lymphbahn durch einen Massageeffekt angeregt. Unter einer Mikrobewegung ist insbesondere eine Bewegung im Bereich von 0,5 mm bis 2,5 mm, insbesondere im Bereich von 1 mm bis 2 mm zu verstehen.

Die an der Innenseite des Bekleidungsstücks angeordneten Vorsprünge weisen eine höhere Festigkeit auf als der Grundkörper des Bekleidungsstücks. Alternativ können die Vorsprünge und der Grundkörper dieselbe Festigkeit aufweisen. Alternativ können die Vorsprünge weicher sein als der Grundkörper. Das Festigkeitsverhältnis von Vorsprüngen zu Grundkörper hängt insbesondere von dem Material des Grundkörpers ab.

Als Innenseite des Bekleidungsstücks ist die Seite des Bekleidungsstücks zu verstehen, welche im getragenen Zustand dem Körper eines Trägers zugewandt ist.

Die Vorsprünge des Bekleidungsstücks sind vorzugsweise punktförmig ausgestaltet. In anderen Worten weisen die Vorsprünge vorzugsweise einen kreisförmigen Querschnitt auf und sind als Teil einer Kugelsphäre ausgestaltet. Alternativ kann der Vorsprung andere Formen, wie beispielsweise einen Pyramidenstumpf sowie jegliche erdenkliche dreidimensionale Form aufweisen. Die Vorsprünge können insbesondere ein Verhältnis Breite zu Länge von 10 zu 1, insbesondere 8 zu 1, insbesondere 6 zu 1, insbesondere 4 zu 1, insbesondere 2 zu 1, insbesondere im Wesentlichen 1 zu 1 aufweisen. Die Breite und die Länge können hierbei die Ausbreitungen der Vorsprünge auf dem Grundkörper des Bekleidungsstücks sein. Unter Breite kann die längste Erstreckung der Vorsprünge gesehen werden. Unter Länge kann die Erstreckung verstehen werden, welche senkrecht zur Breite verläuft. Unter "im Wesentlichen" ist hierbei zu verstehen, dass das Verhältnis aufgrund von Fertigungstoleranzen minimal von einem Verhältnis 1 zu 1 abweichen kann. Die Vorsprünge können insbesondere einen symmetrischen Querschnitt aufweisen. Eine bevorzugte punktförmige Ausgestaltung erhöht den Tragekomfort und ermöglicht eine sanfte Massage des Körperbereichs des Trägers durch die Mikrobewegung der Vorsprünge.

Der Körperbereich, an welchem das Bekleidungsstück vom Träger getragen wird, kann beispielsweise der Unterkörper oder der Oberkörper sein. Das Bekleidungsstück kann am gesamten Unterkörper oder am gesamten Oberkörper getragen werden. Das Bekleidungsstück kann alternativ nur Teile des Unterkörpers oder des Oberkörpers getragen werden. Das Bekleidungsstück wird insbesondere an jenen Körperteilen getragen, an welchen eine Anregung des Lymphflusses notwendig ist.

Ein Bekleidungsstück nach Anspruch 2 stellt eine bevorzugte Ausführung des Grundkörpers dar. Die Ausführung des Grundkörpers als Gewebe bewirkt einen hohen Tragekomfort. Ferner stellt ein Gewebe ein atmungsaktives Material dar.

Ein Bekleidungsstück nach Anspruch 3 regt den Lymphfluss weiter an. Durch den Kompressionsdruck zwischen 2,4 kPa (Kompressionsklasse CCL1) und 4,3 kPa (Kompressionsklasse CCL2) wird der Lymphfluss zusätzlich durch die Kompression des Körperteils angeregt.

Der Kompressionsdruck zwischen 2,4 kPa und 4,3 kPa erhöht außerdem die Massagewirkung der Vorsprünge bei der Mikrobewegung. Es wird insbesondere durch den Kompressionsdruck sichergestellt, dass die Vorsprünge in den Körperbereich des Trägers eingedrückt werden.

Ein Bekleidungsstück nach Anspruch 4 fördert die Mikrobewegung der Vorsprünge durch die schräg und/oder gegenläufig verlaufenden elastischen Faserzüge.

Ein Bekleidungsstück nach Anspruch 5 stellt sicher, dass das Kleidungsstück sicher am Träger verbleibt. Durch die Halteelemente wird hierbei insbesondere sichergestellt, dass das Bekleidungsstück am Körper nicht verrutscht und somit eine Deckung der Bahnen der Vorsprünge mit den Lymphbahnen des Körpers dauerhaft besteht.

Die Halteelemente können beispielsweise als elastische Gummielemente ausgeführt sein. Alternativ können die Halteelemente nach dem Prinzip eines Gürtels mit einer Gürtelschnalle ausgeführt sein.

Ein Bekleidungsstück nach Anspruch 6 stellt eine bevorzugte Ausgestaltung dar. Durch einen Verlauf der mindestens einen Bahn ausschließlich entlang der mindestens einen Lymphbahn wird sichergestellt, dass jeder Vorsprung tatsächlich den Lymphfluss eines Körpers anregt. Ebenfalls wird der Tragekomfort des Bekleidungsstücks erhöht, da die Anzahl der Vorsprünge, welche ein Druckgefühl auf den Körper auswirken können, reduziert werden kann.

Ein Bekleidungsstück gemäß Anspruch 7 ermöglicht ein einfaches Einbringen der Vorsprünge in einen Grundkörper, welcher ein Gewebe umfasst.

Ein Bekleidungsstück nach Anspruch 8 ermöglicht ein nachträgliches Aufbringen von Vorsprüngen auf ein Bekleidungsstück. Durch ein derart nachträgliches Aufbringen ist es beispielsweise möglich, den Grundkörper des Bekleidungsstücks allgemein in den herkömmlichen Größen zu fertigen und die Anordnung der Bahnen nachträglich an die Körperform eines Trägers, insbesondere an den Verlauf der Lymphbahnen eines Trägers, anzupassen.

Ein Bekleidungsstück nach Anspruch 9 stellt ein Bekleidungsstück mit optimaler Festigkeit der Vorsprünge dar. Kunststoff und/oder Silikon weist eine für die Vorsprünge geeignete Festigkeit auf. Ferner sind Kunststoffe oder Silikon hautverträglich und führen nicht zu Reizungen des Trägers.

Ein Bekleidungsstück mit einer Höhe der Vorsprünge gemäß Anspruch 10 hat sich als besonders wirksam erwiesen. Die Höhe der Vorsprünge stellt hierbei ebenfalls die Eindrücktiefe in den Körper des Trägers dar. Die Höhe der Vorsprünge liegt im Bereich zwischen 0,5 mm und 2 mm, insbesondere im Bereich 0,75 mm bis 2 mm, insbesondere im Bereich 1 mm bis 2 mm, insbesondere im Bereich 1,25 mm bis 2 mm, insbesondere im Bereich 1,5 mm bis 2 mm.

Die Höhe der Vorsprünge ist hierbei als Ausdehnung senkrecht zur Hautoberfläche des Trägers zu sehen.

Ein Bekleidungsstück mit Vorsprüngen mit einem Durchmesser gemäß Anspruch 11 hat sich als besonders wirksam bei Anregung des Lymphflusses herausgestellt. Der Durchmesser liegt hierbei im Bereich zwischen 0,5 mm und 3 mm, insbesondere im Bereich 1 mm bis 3 mm, insbesondere im Bereich 1,5 mm bis 3 mm, insbesondere im Bereich 2 mm bis 3 mm.

Der Durchmesser bezieht sich, insbesondere bei nicht kreisförmigen Querschnitten, auf den Umkreis um eine Querschnittsfläche der Vorsprünge, wobei der Umkreis die Querschnittsfläche der Vorsprünge nicht schneidet und mindestens in zwei Punkten berührt.

Ein Bekleidungsstück mit einem Abstand der Vorsprünge gemäß Anspruch 12 hat sich als besonderes effizient bei Anregung des Lymphflusses bewährt. Durch den Abstand wird sichergestellt, dass der Lymphfluss entlang der gesamten Lymphbahn und nicht nur entlang Teilen der Lymphbahn angeregt wird. Der Abstand der Vorsprünge entlang der mindestens einen Bahn liegt hierbei im Bereich von 0,5 cm bis 1,5 cm, insbesondere im Bereich 0,75 cm bis 1,5 cm, insbesondere im Bereich 1 cm bis 1,5 cm. Besonders bevorzugt sind die Vorsprünge gleichmäßig mit einem Abstand von 1 cm zueinander entlang einer Bahn angeordnet.

Ein Bekleidungsstück gemäß Anspruch 13 stellt sicher, dass die Knotenbahnen dauerhaft entlang der Lymphbahnen verlaufen. Hierfür sind die Pufferzonen an den Gelenken des Trägers angeordnet. Diese Pufferzonen sind aus einem elastischen Material gefertigt und kompensieren die Gelenkbewegung des Trägers. Hierdurch wird erreicht, dass die Bahnen mit den Vorsprüngen außerhalb der Pufferzonen auch bei größeren Bewegungen des Trägers sicher entlang der Lymphbahnen verlaufen. Ohne derartige Pufferzonen könnten die Bahnen mit den Vorsprüngen verrutschen und den Lymphfluss nicht mehr effektiv anregen.

Bevorzugt können die Pufferzonen aus einem anderen Material wie der Grundkörper gefertigt sein. Bevorzugt können die Pufferzonen ein höheres Elastizitätsmodul als der Grundkörper aufweisen. Bevorzugt können die Pufferzonen aus einem Gummi-Textil-Mischgewebe gefertigt sein. Alternativ können die Pufferzonen aus Gummi gefertigt sein. Alternativ können die Pufferzonen aus einem elastischen Textilgewebe gefertigt sein. Bevorzugt können die Pufferzonen mit einer verstärkten Strick-/Gewirktechnik mit beigemischten Gummianteilen gefertigt sein.

Ausführungsbeispiele der Erfindung werden nachfolgend anhand der Zeichnungen näher erläutert. Die Figuren zeigen:
- Fig. 1: schematische Darstellung eines als Hose ausgeführten ersten Ausführungsbeispiels eines Bekleidungsstücks mit schematisch gekennzeichneten Bahnen in einer ersten Ansicht,
- Fig. 2: schematische Darstellung des Kleidungsstücks der Figur 1 in einer zweiten Ansicht,
- Fig. 3: eine Ausschnittsvergrößerung III des in Figur 1 schematisch dargestellten Bekleidungsstücks,
- Fig. 4: einen schematischen Querschnitt entlang der in Figur 3 dargestellten Linie IV-IV,
- Fig. 5: schematische Darstellung eines als Armstulpe ausgeführten zweiten Ausführungsbeispiels des Bekleidungsstücks mit schematisch gezeichneten Bahnen in einer ersten Ansicht,
- Fig. 6: den schematischen Verlauf von Bahnen mit Vorsprüngen am Übergang des Oberarms zur Schulter aus einer Rückansicht.

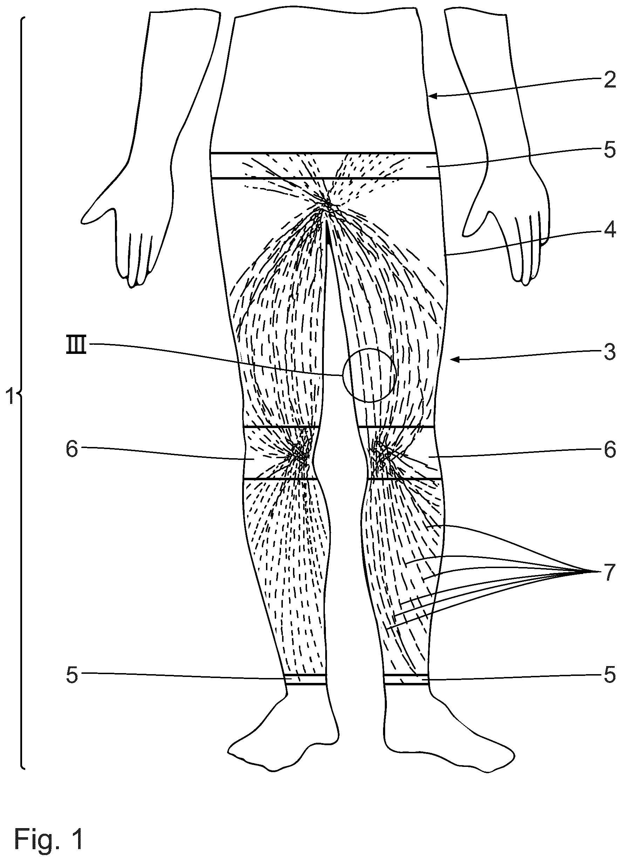

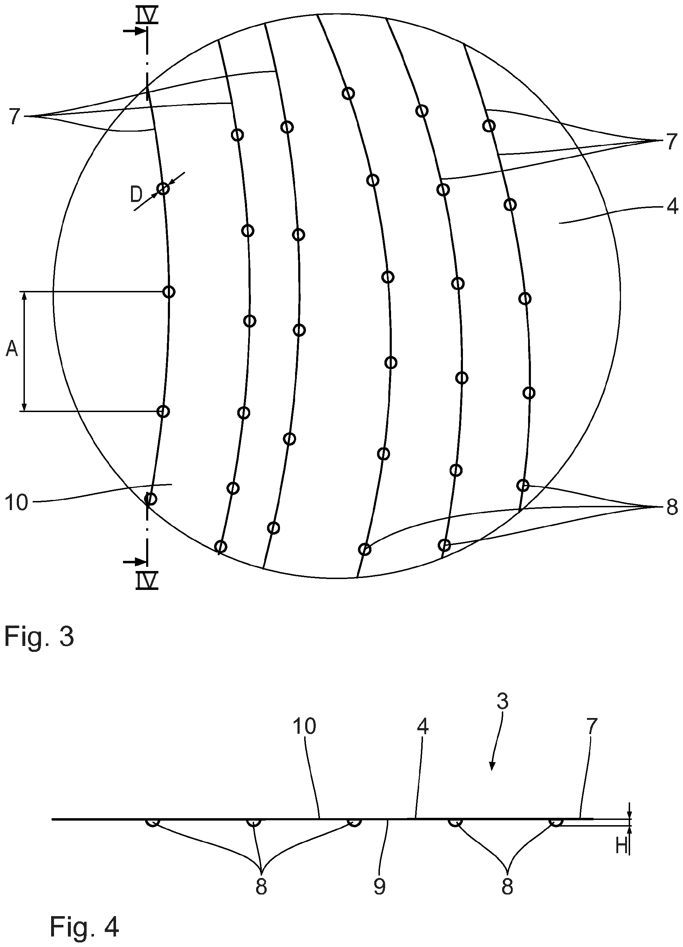

Die Fig. 1 zeigt eine Vorderansicht eines Körperbereichs 1 eines Trägers 2, welcher ein Bekleidungsstück 3 trägt. Das Bekleidungsstück 3 umfasst einen Grundkörper 4 mit Halteelementen 5 und Pufferzonen 6. Auf dem Kleidungsstück 3 verlaufen schematisch eingezeichnete Bahnen 7, auf welchen Vorsprünge 8 angeordnet sind. Die Vorsprünge 8 sind insbesondere aus Fig. 3 und 4 ersichtlich. Das Bekleidungsstück 3 weist eine dem Körperbereich 1 des Trägers 2 zugewandte Innenseite 9 und eine dem Körperbereich 1 des Trägers 2 abgewandte Außenseite 10 auf. Die Innenseite 9 ist insbesondere in Fig. 4 ersichtlich.

Im vorliegenden Beispiel ist das Bekleidungsstück 3 eine Hose. Das Bekleidungsstück 3 wird durch die Halteelemente 5 sicher an einem Träger gehalten und ein Verrutschen des Bekleidungsstücks 3 wird verhindert. Die Halteelemente können beispielsweise als elastische Gummibänder oder als Gürtel mit Gürtelschnalle ausgeführt sein.

Die Bahnen 7 des Bekleidungsstücks 3 verlaufen entlang den nicht gezeigten Lymphbahnen des Körperbereichs 1 des Trägers 2. Die Bahnen 7 verlaufen hierbei entlang mindestens einer Lymphbahn des Körperbereichs 1 des Trägers 2, bevorzugt entlang sämtlicher Lymphbahnen des Körperbereichs 1 des Trägers 2.

Die Pufferzonen 6 sind im Bereich der Gelenke des Trägers angeordnet. Die Pufferzonen 6 des Bekleidungsstücks 3 sind aus einem elastischen Material ausgeführt. Durch die elastischen Pufferzonen 6 kann gewährleistet werden, dass sich die Bahnen 7 bei Bewegungen des Trägers 2 nicht von den Lymphbahnen des Trägers weg verschieben.

Der Grundkörper 4 des Bekleidungsstücks 3 ist auf einem Gewebe gefertigt. Das Gewebe bewirkt einen Kompressionsdruck zwischen 2,4 kPa und 4,3 kPa auf den Körperbereich 1 des Trägers 2. Durch den Kompressionsdruck werden die Vorsprünge 8 in den Körperbereich 1 des Trägers 2 gedrückt. Ebenfalls wird durch den Kompressionsdruck der Lymphfluss angeregt.

Bei Bewegung des Trägers 2 erfahren die Vorsprünge 8, welche auf der Innenseite 9 des Bekleidungsstücks 3 entlang der Bahnen 7 angeordnet sind, Mikrobewegungen im Bereich < 1 mm. Durch die Mikrobewegung der Vorsprünge 8 wird der Lymphfluss im Körperbereich 1 des Trägers 2 angeregt.

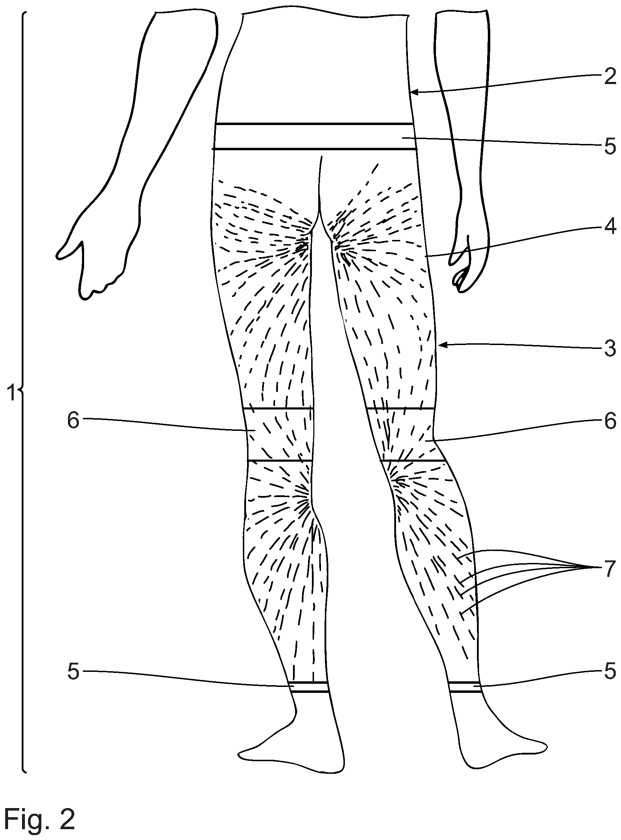

Fig. 2 zeigt den schematisch dargestellten Verlauf der Bahnen 7 des Bekleidungsstücks 3 in einer Rückansicht des Trägers 2.

Die Fig. 3 zeigt eine Detailansicht des mit III markierten Bereichs der Fig. 1. In der Detailansicht ist ein Bereich des Grundkörpers 4 gezeigt, auf welchem die Bahnen 7 eingezeichnet sind. Die Bahnen 7 sind lediglich zur Orientierung eingezeichnet und an einem tatsächlichen Bekleidungsstück 3 lediglich anhand der Vorsprünge 8 erkennbar. Ebenfalls sind die Vorsprünge 8 auf der Außenseite 10 des Bekleidungsstücks 3 eingezeichnet. Die Vorsprünge 8 sind jedoch lediglich durch deren Kontur auf der Außenseite erkennbar.

Die Vorsprünge 8 weisen im vorliegenden Beispiel einen kreisförmigen Querschnitt mit einem Durchmesser D auf. Der Durchmesser D ist insbesondere kleiner als 3 mm. Generell liegt der Durchmesser D im Bereich von 0,5 mm bis 3 mm. Die Vorsprünge 8 weisen innerhalb einer Bahn 7 einen Abstand A zueinander auf. Der Abstand A beträgt maximal 1,5 cm. Der Abstand A liegt insbesondere im Bereich 0,5 cm bis 1,5 cm.

In Fig. 4 ist eine Darstellung entlang der in Fig. 3 dargestellten Linie IV-IV gezeigt. Wie aus Fig. 4 erkenntlich, sind die Vorsprünge 8 an einer Innenseite 9 des Bekleidungsstücks 3 angeordnet. Die Innenseite 9 ist dem Körperbereich 1 des Trägers 2 zugewandt.

Die Vorsprünge 8 weisen eine Höhe H auf. Die Höhe H beträgt maximal 2 mm. Insbesondere liegt die Höhe H der Vorsprünge 8 im Bereich 0,5 mm bis 2 mm.

Die Vorsprünge 8 können auf verschiedene Weisen in den Grundkörper 4 des Bekleidungsstücks 3 eingebracht werden. Die Vorsprünge 8 können beispielsweise durch eine Stick- oder Knotentechnik in den Grundkörper eingebracht werden. Alternativ können die Vorsprünge 8 durch Aufkleben von Erhöhungen an den Grundkörper 4 angebracht werden. Hierbei können die Vorsprünge 8 beispielsweise aus Kunststoff oder Silikon bestehen.

Das schematisch als Hose dargestellte Bekleidungsstück 3 kann ebenfalls als jegliches beliebiges Kleidungsstück ausgeführt sein. Das Bekleidungsstück 3 kann beispielsweise als T-Shirt, Strumpf, Pullover, Ellenbogenschoner oder ähnliches ausgeführt sein.

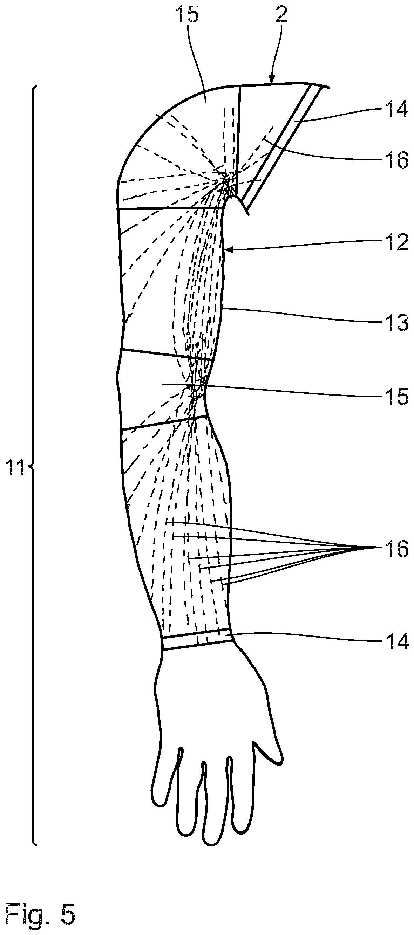

Fig. 5 zeigt einen Körperbereich 11 eines Trägers 2. Der Körperbereich 11 ist hierbei der Arm eines Trägers 2. Am Körperbereich 11 ist ein Bekleidungsstück 12 angeordnet, welches als Armstulpe ausgeführt ist. Das Bekleidungsstück 12 umfasst einen Grundkörper 13, Halteelemente 14, Pufferzonen 15 sowie Bahnen 16. Entlang der Bahnen 16 sind ebenfalls nicht gezeigte Vorsprünge 8 angeordnet.

Wie bereits im ersten Ausführungsbeispiel dienen die Halteelemente 14 dazu, dass das Bekleidungsstück 12 bei Bewegungen nicht verrutscht. Die Pufferzonen 15 stellen sicher, dass Bewegungen kompensiert werden können, damit die Bahnen 16 auch bei Bewegungen entlang der Lymphbahnen des Trägers 2 verlaufen.

Das Bekleidungsstück 12 unterscheidet sich vom Bekleidungsstück 3 insbesondere darin, dass dieses einen anderen Körperbereich 11 abdeckt. Die Funktion der einzelnen Bestandteile des Bekleidungsstücks 12 ist mit der Funktion der Bestandteile des Bekleidungsstücks 3 gleichzusetzen.

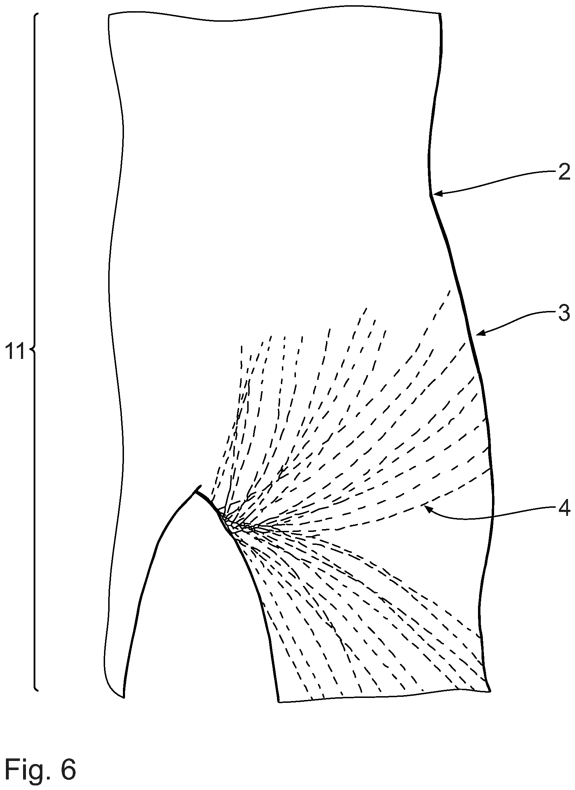

Fig. 6 zeigt schematisch den Verlauf der Bahnen 16 im Schulterbereich aus einer Ansicht von hinten.

## Patentansprüche

1. Bekleidungsstück (3, 12) zum Anregen eines Lymphflusses in einem Körperbereich (1, 11) eines Trägers (2) mit
einem Grundkörper (4, 13) aufweisend
eine Vielzahl an Vorsprüngen (8), welche an einer Innenseite (9) des Bekleidungsstücks (3, 12) angeordnet sind,
wobei die Vorsprünge (8) derart angeordnet sind, dass diese mindestens eine Bahn (7, 16) ausbilden,
wobei die mindestens eine Bahn (7, 16) entlang mindestens einer Lymphbahn eines Trägers (2) verläuft.

2. Bekleidungsstück (3, 12) nach Anspruch 1, **dadurch gekennzeichnet, dass** der Grundkörper (4, 13) ein Gewebe ist.

3. Bekleidungsstück (3, 12) nach einem der Ansprüche 1 oder 2, **dadurch gekennzeichnet, dass** der Grundkörper (4, 13) einen Kompressionsdruck zwischen 2,4 kPa und 4,3 kPa auf den Körperbereich (1, 11) des Trägers (2) ausübt.

4. Bekleidungsstück (3, 12) nach einem der vorhergehenden Ansprüche, **dadurch gekennzeichnet, dass** der Grundkörper (4, 13) schräg und/oder gegenläufig verlaufende elastische Faserzüge umfasst.

5. Bekleidungsstück (3, 12) nach einem der vorhergehenden Ansprüche, **dadurch gekennzeichnet, dass** der Grundkörper (4, 13) Halteelemente (5, 14) zum Halten des Bekleidungsstücks (3, 12) an einem Körperbereich (1, 11) des Trägers (2) aufweist.

6. Bekleidungsstück (3, 12) nach einem der vorgehenden Ansprüche, **dadurch gekennzeichnet, dass** die mindestens eine Bahn (7, 16) ausschließlich entlang mindestens einer Lymphbahn des Trägers (2) verläuft.

7. Bekleidungsstück (3, 12) nach einem der vorgehenden Ansprüche, **dadurch gekennzeichnet, dass** die Vorsprünge (8) durch Stick- oder Knotentechnik in den Grundkörper (4, 13) eingebracht sind.

8. Bekleidungsstück (3, 12) nach einem der vorgehenden Ansprüche, **dadurch gekennzeichnet, dass** die Vorsprünge (8) durch Aufkleben an dem Grundkörper (4, 13) angebracht sind.

9. Bekleidungsstück (3, 12) nach einem der vorgehenden Ansprüche, **dadurch gekennzeichnet, dass** die Vorsprünge (8) aus Kunststoff und/oder Silikon bestehen.

10. Bekleidungsstück (3, 12) nach einem der vorgehenden Ansprüche, **dadurch gekennzeichnet, dass** die Vorsprünge (8) eine Höhe (H) zwischen 0,5 mm und 2 mm aufweisen.

11. Bekleidungsstück (3, 12) nach einem der vorgehenden Ansprüche, **dadurch gekennzeichnet, dass** die Vorsprünge (8) einen Durchmesser (D) zwischen 0,5 mm und 3 mm aufweisen.

12. Bekleidungsstück (3, 12) nach einem der vorgehenden Ansprüche, **dadurch gekennzeichnet, dass** die Vorsprünge (8) entlang der mindestens einen Bahn (7, 16) einen Abstand (A) von 0,5 cm bis 1,5 cm zueinander aufweisen.

13. Bekleidungsstück (3, 12) nach einem der vorgehenden Ansprüche, **dadurch gekennzeichnet, dass** das Bekleidungsstück (3, 12) elastische Pufferzonen (6, 15) aufweist.
